# EUROPEAN PATENT APPLICATION

(11) **EP 3 232 354 A1**
(43) Date of publication of application: **18.10.2017**
(21) Application number: 17165632.5
(22) Date of filing: 07.04.2017
(51) Int. Cl.: G06F 19/00

(54) **SYSTEM AND METHOD TO CREATE, MONITOR, AND ADAPT INDIVIDUALIZED MULTIDIMENSIONAL HEALTH PROGRAMS**

(30) Priority: 15.04.2016 US 201615130770
(71) Applicant: Palo Alto Research Center Incorporated, Palo Alto, California 94034 (US)
(72) Inventor: RAM, Ashwin, Palo Alto, CA 94306 (US); YOUNGBLOOD, Gregory Michael, San Jose, CA 95123 (US); NELSON, Lester D., Santa Clara, CA 95050 (US); VENKATAKRISHNAN, Anusha, Menlo Park, CA 94132 (US); PIROLLI, Peter L., San Francisco, CA 94116 (US); SILVA, Michael K., Jr., Fairfield, CA 94533 (US); MOHAN, Shiwali, Palo Alto, CA 94306 (US)
(74) Representative: Gill Jennings & Every LLP

(57) **Abstract**

A method and system for delivering a multidimensional wellness coaching program and recommendation to a computing device. During operation, the system receives a request for content to assist the user in achieving a health and wellness goal from the computing device. The system estimates numeric values for a radar chart. The system may create a customized program by combining components and primitives and the customized program includes a set of activities for the user to perform. The system may receive user input and sensor and user activity data from the computing device. Based on the received information, the system may modify the radar chart. The system may modify components of the customized program based on the radar chart. The system may generate a coaching recommendation based on the radar chart. The system then sends the customized program and coaching recommendation to the computing device.

## Description

### Field

The present disclosure generally relates to personalized health and wellness coaching. More specifically, the present disclosure relates to a method and system for coaching users using customized health programs while assessing and visualizing their progress towards health goals along multiple health and wellness dimensions.

### Related Art

Persons living their daily lives are often are either consistently engaged in less than healthy activities or consistently lacking in activities that promote their health and wellbeing. These people are often at increased risk for the development of future health problems or decreased quality of life. For example, the National Center for Chronic Disease Prevention and Health Promotion states that:
*Health risk behaviors are unhealthy behaviors you can change. Four of these health risk behaviors-lack of exercise or physical activity, poor nutrition, tobacco use, and drinking too much alcohol-cause much of the illness, suffering, and early death related to chronic diseases and conditions.*

Intervening in a person's daily life on such issues brings with it challenges. For example, dealing with one or a limited set of behaviors is not in itself sufficient to affect those few attributes or on the whole of a person's wellbeing. Also, these are long-term circumstances, where it is unreasonable to expect that a person's interests, motivations, and resources will remain stable over the long term.

There are many programs, applications, and wearable devices to help users monitor and improve health and wellness. However, they address only one or two aspects of individual health. Some examples are Mayo Clinic diet (nutrition), RunKeeper (cardio), Headspace (stress), Jillian Michaels (cardio and strength), Yoga (flexibility and stress), Fitbit (cardio and sleep), and Aura (sleep). An individual must monitor, manage and coordinate multiple programs and recommendations to manage chronic or pre-chronic conditions or meet guidelines for healthy living, which is a complex task that creates cognitive overload.

Moreover, such single program or approaches tends to focus on results over a specific shorter period of time (e.g., weeks and months). Some parties recognize the need to attempt to define a "maintenance phase" (e.g., Medifast for nutrition) extending a single program or approach for an indefinite period but do not recognize a person's evolving interests and needs. An individual must monitor, manage and coordinate multiple programs over the course of longer periods (e.g., months and years).

Current health programs are predesigned by subject matter experts (SMEs). This includes programs offered by providers and payers (e.g., Palo Alto Medical Foundation (PAMF) weight management, Centers For Disease Control and Prevention (CDC) prediabetes coaching, National Health Service (NHS) couch to 5k), employers (e.g., Global Corporate Challenge, General Motors diet), and popular programs (e.g., Weight Watchers, Jillian Michaels). These one-size-fits-all programs are not customizable to individual users, resulting in lower efficacy and higher dropout rates.

### SUMMARY

One embodiment of the present invention provides a method for delivering a multidimensional wellness coaching program and recommendation to a computing device (e.g., mobile device) belonging to a user. During operation, the system receives a request from the computing device for content to assist the user in achieving a health and wellness goal. The system then estimates a set of a numeric values for a plurality of dimensions of a radar chart for the user. The system may create a customized program for the user by combining components and primitives based on the plurality of dimensions of the radar chart. The customized program includes a set of activities for the user to perform. The system may then receive user input from the computing device. The system may then receive sensor data and user activity data from the computing device. Based on the sensor data, user input, and user activity data, the system may modify one or more of the numeric values for the plurality of dimensions of the radar chart. The system may modify one or more components of the customized program based on the set of numeric values for the plurality of dimensions of the radar chart. The system may then generate a coaching recommendation based on the set of numeric values for the plurality of dimensions of the radar chart. The system then sends the customized program and coaching recommendation to the computing device.

In one variation on this embodiment, the system may generate a suggested goal along a particular dimension for the user using data obtained from a user model and a program received from a provider and deliver the suggested goal to the user.

In a further variation on this embodiment, the system may display a visual representation of the user's health goal at a particular moment in time, including displaying a visual representation of the radar chart that includes five dimensions of strength, nutrition, aerobic, relaxation, and flexibility for the user's current health condition.

In a further variation on this embodiment, the system may analyze activities, conversations, and interactions of the user with the teams that the user participates in, and modify a user model based on the analysis.

In a further variation on this embodiment, creating the customized program for the user further includes selecting components and/or primitives for creating the customized program from a collection of components and/or primitives that cover all dimensions of the health chart while varying the emphasis on each dimension depending on the current user condition.

In a further variation on this embodiment, the system receives a program from a program provider and determines a plurality of constituent components from the program.

In a further variation on this embodiment, the system assesses a user's progress along one or more dimensions towards a particular goal. The system may then determine that the user has regressed along a particular dimension of the radar chart according to a regression rule. The system may generate additional coaching recommendations for the user, based on the numeric value for the particular dimension of the radar chart and the regression rule. The system may deliver the additional coaching recommendations to the computing device.

In a further variation on this embodiment, the system obtains information from a user model. Based on information from the user model, the system may generate an intervention along a particular dimension that suggests the user perform an action. The system may then deliver the intervention to the computing device.

In a further variation on this embodiment, receiving the request from the computing device further includes receiving information indicating at least a current user condition and estimating the set of numeric values further includes estimating the set of numeric values based on the received information indicating at least the current user condition.

### BRIEF DESCRIPTION OF THE FIGURES

FIG. 1 presents a diagram illustrating an exemplary radar chart for a five dimensional challenge, in accordance with an embodiment of the present invention.
FIG. 2 presents a diagram illustrating an exemplary multidimensional health and wellness system, in accordance with an embodiment of the present invention.
FIG. 3 illustrates an exemplary screenshot of users participating in challenges in small teams.
FIG. 4 illustrates an exemplary screenshot of an iPhone application that users may use to report on challenge activities.
FIG. 5 illustrates an exemplary screenshot of an iPhone application that users may use to engage with teammates on challenge activities and be social.
FIG. 6A and 6B presents a flow chart illustrating an exemplary process for delivering a wellness coaching program and recommendation to a mobile device belonging to a user, in accordance with an embodiment of the present invention.
FIG. 7 presents a block diagram illustrating an exemplary health and wellness apparatus, in accordance with an embodiment of the present invention.
FIG. 8 presents a server in a multidimensional health and wellness system, in accordance with an embodiment of the present invention.

In the figures, like reference numerals refer to the same figure elements.

### DETAILED DESCRIPTION

The following description is presented to enable any person skilled in the art to make and use the invention, and is provided in the context of a particular application and its requirements. Various modifications to the disclosed embodiments will be readily apparent to those skilled in the art, and the general principles defined herein may be applied to other embodiments and applications without departing from the spirit and scope of the present invention. Thus, the present invention is not limited to the embodiments shown, but is to be accorded the widest scope consistent with the principles and features disclosed herein.

The data structures and code described in this detailed description are typically stored on a computer-readable storage medium, which may be any device or medium that can store code and/or data for use by a computer system. The computer-readable storage medium includes, but is not limited to, volatile memory, non-volatile memory, magnetic and optical storage devices such as disk drives, magnetic tape, CDs (compact discs), DVDs (digital versatile discs or digital video discs), or other media capable of storing computer-readable media now known or later developed.

### Overview

Embodiments of the present invention solve the problems of cognitive overload, maintenance, and uncustomizable programs by coaching users to manage their health and wellness using a holistic, multidimensional approach. A health and wellness system uses a multidimensional indicator, referred to as a radar chart, to represent multiple health and wellness dimensions in a holistic manner that is intuitive for humans. These dimensions may include, for example, strength, nutrition, aerobic, relaxation, and flexibility. The system may help an individual user monitor and visualize their status and progress along each dimension. The system may continuously update the multidimensional indicator based on an assessment of the user's progress. The system also includes techniques to automatically create customized multidimensional health programs, including goals, recommendations and coaching interventions, for each user to provide coverage of appropriate dimensions at appropriate times. The system may dynamically combine components to customize programs for each user and adapt the programs to individual users. The system may also constantly assess and reassess the user's health and wellness condition to determine the user's progression and/or regression, in order to deliver focused content to the user's computing device that is better adapted for the user.

### Rationale for Health And Wellness Design

The rationale for the health and wellness design presented in this disclosure builds on the observation that long-term health and wellness depends in large part on a person maintaining sustained healthy activities over extended periods of time, and many times through an entire lifetime. This disclosure assumes that a health and wellness intervention program is designed with proper physiological expertise needed to define activities that are physically sustainable in terms of nutrition and physical activity. This disclosure further assumes that such a program is about a lifestyle and other attributes and dimensions of behavior and hence involves much more than weight management alone.

### Health Radar

This disclosure describes a "health radar" encoding multiple "health dimensions" that a multidimensional health and wellness system can visualize in a radar chart. A radar chart is one example of a multi-dimensional representation of a health program. Other kinds of visualization may include matrices, grids, bar charts, pie charts, scatter plots, and histograms. The health radar may include five dimensions that are part of most accepted health guidelines: nutrition, cardio (or aerobic), strength, flexibility, and stress (or relaxation). The health radar can be extended to include other dimensions that are relevant to specific health scenarios. Each health dimension includes several "health primitives" which are activities designed to create and reinforce healthy habits along that dimension. The system may use a set of artificial intelligence techniques, together comprising an automated "health coach," to model an individual's status along these dimensions and create a personalized health coaching program designed to help the individual adopt and maintain a healthy lifestyle. The techniques are guided by "health coaching policies" that encode how programs should be adapted and varied across days, weeks and months based on the individual's health status, short- and long-term progress, preferences, and contextual constraints.

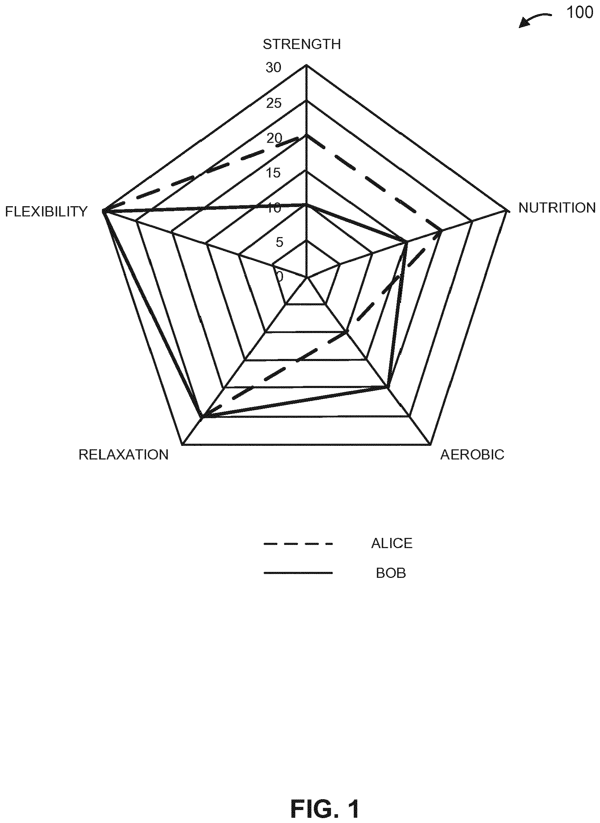

The system may use the health radar to address a person's evolving interests and needs by using "paths" through the points of the radar to engage a person over longer periods of time. For example, with respect to the health radar shown in FIG. 1, for some number of weeks a person may be addressing each endpoint but paying particular attention to cardio in preparation for an upcoming event (e.g., a "Walk-a-Thon" for a favored charity). After the event, the focus may return to flexibility and nutrition for a time. This process can be repeated over and over, but in the longer term, help is needed to achieve the overall balance across all the points.

### Health Radar Challenge

There are a number of examples of how wellness interventions are not proving sustainable. Often, users try various new interventions, but once the novelty wears off, if the intervention fails to engage users, they get bored and eventually give up (e.g., wearable trackers such as Fitbit). The other end of this continuum is where users successfully establish a habit through an intervention, but given that there is not long-term maintenance, users can easily rebound and break out of a healthy habit. This problem compounds when users try serial interventions due to lack of success with single interventions. But, when habit acquisition is incomplete, and the intervention does not focus on maintenance, serial rebounding can cause even more frustration to the user.

To avoid these problems, the inventors propose a health and wellness program that is designed as a series of things to do, that reinforce prior activities, that the user can keep doing, and with the activities evolving over time.

This disclosure describes an approach that organizes programs called challenges that are designed to guide people towards healthier living. A challenge has participants perform specific activities and report on how they did with these activities. People may join with others (e.g., teams) to participate in a challenge together to communicate and share resources (e.g., using mobile devices and applications) to facilitate their daily health challenge activities. For example, the NutriWalking challenge gives people a progression of nutrition habits to learn and walking activities to perform over the course of 8 weeks.

Specifically, a challenge is defined as an ordered set of activities and related descriptive content that when performed are expected to positively impact a health/wellness outcome. Further, the actions a person takes to perform or participate in each activity should be well defined in terms of who, what, when, where, how, how much, and possibly why a person would do each action. The challenge content should provide such well defined descriptions through persistently available media or messaging.

### Defining a Radar Challenge

A challenge is a "health radar challenge" (hereafter called a radar challenge) if it has the following five attributes:
1. Systemic: The health and wellness problem that the challenge is meant to address is a systemic problem requiring a systemic solution. Challenge activities and their related content and messaging are the primary components of an intervention solution. In a radar challenge these activities should address a range of health/wellness interventions that are systemically interrelated to the outcome. In other words, a radar is multi-faceted along several dimensions. For example, as a society we gain a pound a year, leading to obesity. As was noted above, obesity intervention is multi-dimensional and the activities defined for a challenge should reflect contribution in these dimensions. Different challenge dimensions might include for example, nutrition and physical activity, as both are needed for a wellness outcome. Note that an individual activity might have contributions across dimensions (e.g., pilates exercise might contribute both to strength outcomes as well as flexibility and balance).
2. Interchangeable: The solution components expressed in the challenge are to some degree interchangeable within a dimension. At any given period of time when an activity is supposed to be performed (e.g., today, this week) another (or a small set) can be performed and have beneficial impact on the overall health/wellness outcome desired. For example, it may be raining today, so one can perform an activity of stair climbing instead of taking a walk. Alternatively, on a weekly time scale, a user may consider eating vegetable soups this week instead of the usual salads for lunch.
   Note that the interchangeable attribute is related to concepts of progression and regression. Progression means that the user is moving forward towards his goal, and regression means that the user is moving backwards, in terms of working towards his goal. For example, a user may be walking and progressing to jogging. If the user has trouble jogging then he might regress back to walking.
   In some embodiments, progressing involves removing helpful advice incrementally, and regressing involves incrementally adding more helpful advice to help the user achieve the goal. For example, if the user has a goal of eating slowly, and he has a difficult time achieving that goal, then the system may recommend that the user count the number of bites the user takes in a meal, and try to increase the number of bites over time. This is a scaffolding technique that provides support for the user until the user can move forward without the additional advice. Another example is the system may engage the user's teammates to assist the user in performing an activity if the user encounters difficulty with the activity. In some embodiments, the system may include progression and regression rules that define when the user is progressing or regressing, including progressing or regressing within a dimension.
3. Interleavable: The solution components expressed in the challenge are to some degree interleavable between dimensions. Different dimensions may (or should) be addressed at different times to have a beneficial impact on health/wellness outcome. For example, the system may recommend that the user not strength train two days in a row, and instead mix in cardio activity in between days. Greater emphasis might be placed on one dimension over another in the short term, but the effects across dimensions get balanced over the long run given the longitudinal nature of the radar intervention. For example, one may place a greater emphasis on cardio exercise in preparation for a 10K run next month, and then afterwards things settle down again into a more balanced set of activities. As another example, the system may interleave meditation with stretching and eating specific foods with a set of activities.
4. Spanning: The solution (expressed in terms of activities being asked of people) spans across the identified dimensions. Further, the activities aim to be minimally complete with respect to achieving the outcome and not involve unnecessary demands to achieve a beneficial outcome. For example, the span of interventions aimed at obesity could be assessed by looking at the coverage (and duplicate coverage) of the range of influences as expressed in the Obesity System Influence Diagram.
5. Sustainable: The solution is sustainable. The combined activities are designed to maintain long term benefit to health/wellness outcomes. For example, as a society we gain a pound a year, which leads to obesity. Some people claim that 10,000 steps a day removes a pound a year. Walking is considered a "life long" activity for most. Combined together, this program claims to offer sustainable weight management.

As activities are being considered for inclusion in a new challenge, the system may weigh their contribution to each dimension. Assessing and communicating the sum effect of these activities to challenge participants as they are performed over time is considered in the next section.

### Radar Interaction and Experience Strategy

FIG. 1 presents a diagram illustrating an exemplary radar chart 100 for a five dimensional challenge, in accordance with an embodiment of the present invention. The inventors have chosen the radar chart for visualizing progress on a radar challenge. FIG. 1 depicts an example visualization of the progress of two individuals along multiple dimensions. FIG. 1 illustrates the comparative progress of Alice and Bob along five dimensions of health and wellness outcomes. In some embodiments, the system can determine and update numeric values for each dimension of the radar chart associated with a user. These numeric values may indicate the degree to which a user has made progress along a respective dimension. The radar chart includes attributes such as:
- Multivariate representation: Radar charts express multivariate data such as activity along different dimensions.
- Easy interpretability by users: A radar chart allows a user to grasp and reason based on a naive interaction model of "bigger and rounder is better." For example, Alice can see that that her aerobic progress is lagging behind her progress in the other dimensions.
- Comparison across users: The radar chart shows that Alice and Bob have different strengths and weaknesses. Some embodiments may also include a roundness measure such as roundness = (4*Area*pi)/(Perimeter^2) to assess a person's individual balance across dimensions.
- Comparison over time: By showing the progression of one user over time through animation, a person could get a sense of what was occurring in particular time frames across dimensions.

### Health Primitives

Health primitives encode knowledge about daily nutrition and exercise goals, activity profile information including metabolic equivalents, substitutions, safety, and effective sequences that improve health while building good habits through reinforcement and encouragement. They are represented in a form that can be visualized in an intuitive, human-friendly manner, and automated artificial intelligence techniques for automated health coaching may also use health primitives. Example health primitives are shown below:
(depiction of "add a vegetable" (activity) and associated information, for "eat more vegetables" habit)
(depiction of "walk 30 minutes" (activity) and associated information, for "5 days a week of physical activity" habit)

Health primitives may include activity goals, recommendations, and coaching interventions designed to promote a healthy lifestyle habit along a health dimension. The system may include artificial intelligence planning and scheduling techniques to create personalized health programs by combining health primitives into a systematic program that spans several weeks. The program covers all dimensions of the health radar, placing different emphasis on each dimension depending on the individual's status and need.

Health primitives can be represented using a frame:

| Name | |
|---|---|
| Description (how to do it) | |
| Benefit (the goal of doing it) | |
| Explanation (why it's good for you or why you should do it) | |
| Progression (how to step it up when the user has mastered it) | |
| Regression (how to tone it down when the user regresses) | |
| Substitutions (an equivalent activity that accomplishes the same health goal) | |
| Problems (what difficulties a user might expect to have when doing it and how to address those problems) | |
| Coaching tips (how to help the user understand what it is, how to do it, and how to keep making progress despite problems) | |

### Exemplary Multidimensional Health and Wellness System

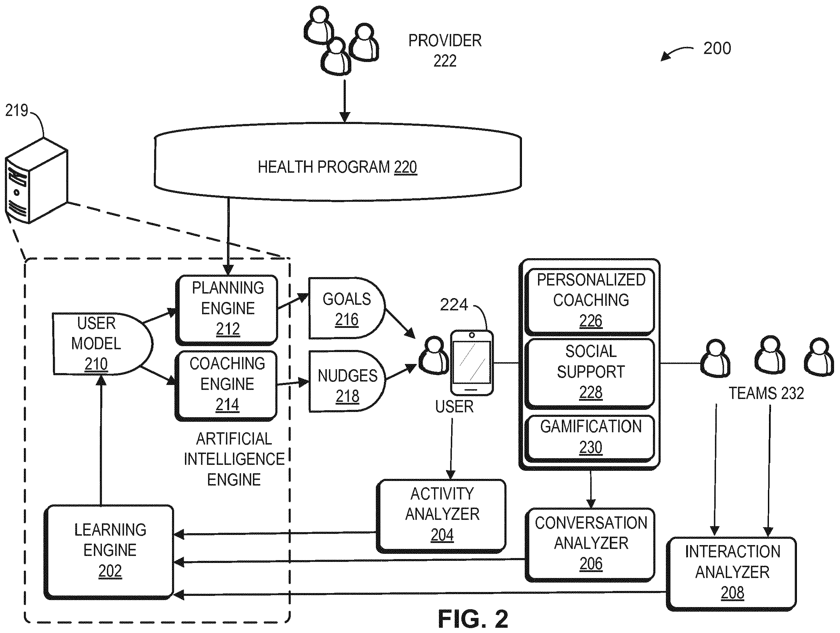

FIG. 2 presents a diagram illustrating an exemplary multidimensional health and wellness system 200, in accordance with an embodiment of the present invention. System 200 may include a learning engine 202 that learns from input received from an activity analyzer 204, a conversation analyzer 206, and an interaction analyzer 208. Learning engine 202 may create and/or update a user model 210. System 200 may include a planning engine 212 and a coaching engine 214 for generating and sending goals 216 and nudges 218, respectively. System 200 may receive a health program 220 from a provider 222 that planning engine 212 uses to generate goals 216. Learning engine 202, user model 210, planning engine 212, and coaching engine 214 may be located on a server 219. System 200 sends the goals and nudges to a mobile device 224 belonging to a user. Mobile device 224 may have a mobile application installed with components such as personalized coaching 226, social support 228, and gamification 230. The user may interact with other teams 232 through the mobile application installed on mobile device 224.

System 200 may be a client/server system which delivers individual customized wellness programs and coaching recommendations to a user on their mobile phone or other computing device. A mobile device is one possible method of delivery, and system 200 may deliver the programs and recommendations to any type of client including mobile devices, web applications running on a personal computer, and large displays such as a display in a home entertainment system. Furthermore, the functionality described herein may be distributed between clients and servers in many ways. For example, the server may perform some computation and communicate to clients such as mobile devices as needed. The client may also perform computations and optionally communicate back to the server, or the client may communicate and share with other clients (e.g., peer-to-peer).

System 200 may implement health coaching policies that create customized goals, recommendations and coaching interventions for an individual on a sub-daily, daily, or weekly basis (or at any appropriate time), based on their health conditions, health goals, recent history, progress, ability, and motivation.

System 200 may use ecological momentary assessment techniques to determine the user's health and wellness and provide coaching advice that allows the user to achieve his goals. System 200 may obtain data in real-time indicating the user's health and wellness and perform an in the moment assessment of the user's condition. This is more effective and accurate than if the user consults with a health practitioner weeks after health events occur. For example, when the user is walking or running, system 200 can assess whether the user is tired in real-time, within a few hours, or a few days, before the event fades from the user's memory. System 200 may analyze the radar chart to determine the differences between the user's current health and wellness condition, and the user's desired target health and wellness condition. Based on the differences, system 200 may determine in the moment tips or advice to deliver to the user's mobile device.

System 200 may assess and reassess the user to determine the user's progression and/or regression along the dimensions of the radar chart. The assessments may affect the activities that the user performs along the multiple dimensions. As the user is performing activities, system 200 may reassess the user to determine whether the user is progressing or regressing, and adjust the messages and activities accordingly. System 200 may perform the assessments using multiple techniques, including questions and answers and sensors. In some embodiments, system 200 may accumulate and assess an individual's health goals and conditions over time to determine the overall balance across the dimensions of the radar chart.

System 200 can assess the user's health and wellness condition by analyzing data received through four sources of input. System 200 obtains data about the user by receiving answers from the user in response to questions presented to the user, examining communications and interaction between the user and other team members, receiving the user's self-reported information (e.g., clicking on selected buttons) including the user's history, and receiving sensor data from the user's mobile device. For example, system 200 can present a question to the user to rate whether an activity was interesting on a scale of 1 to 10.

Learning engine 202 may use a number of techniques, including machine learning techniques, to learn from elements received from the analyzers. The analyzers may be located on user's mobile device and other team member's mobile device, or, in some embodiments, can also be located on the server. The analyzers may collect data on the user and the team as an ongoing process. In some embodiments, the analyzers can filter elements for learning engine 202, and learning engine 202 detects and determines the patterns that exist in the filtered elements.

In some embodiments, activity analyzer 204 can analyze user activity, including analyzing user activity while the user is performing activities. For example, activity analyzer 204 may use statistical pattern matching to analyze the user's activity data to determine that the user is walking. Conversation analyzer 206 may analyze the language used in conversations between the user and team members, and also between other team members. Conversation analyzer 206 may determine which team members are communicating with each other and examine the keywords in the conversations. Conversation analyzer 206 may determine the sentiment and mood, and, in some embodiments, may use keywords to determine the mood of the conversations.

Interaction analyzer 208 may analyze the interactions between the team members, including the user's interaction with other team members, to determine which individuals are interacting with other individuals. Interaction analyzer 208 may also determine how often team members interact with each other to determine the degree of closeness in the bond between the respective team members. For example, discussions and interactions between people with closer bonds are more relevant than interactions between people with lesser bonds and system 200 may store that information in user model 210.

System 200 may generate and/or update user model 210. User model 210 may include data describing the user, such as the activity, conversation, and interaction data received from the analyzers. System 200 uses information from user model 210 to determine the goals and nudges that the artificial intelligence engine generates and delivers to mobile device 224. System 200 may intervene along different dimensions to send appropriate nudges and goals for each dimension to the user.

Planning engine 212 obtains data from user model 210 in order to generate goals 214. Coaching engine 214 obtains data from user model 210 in order to generate nudges 218. Nudges are messages that system 200 sends to the user to attempt to modify the user's behavior. Nudges are one example of interventions. Interventions can also include other examples such as hints, suggestions, recommendations, persuasion, instruction, negotiation, offering alternatives, recognition, rewards, and self-reflection.

Provider 222 is one or more experts that provides a health program. For example, one health program may help the user with diabetes, or another health program may assist the user with walking. System 200 may break down (e.g., separate) a multidimensional health program and determine individual components from the health program. System 200 may use a set of components as building blocks to dynamically generate and customize programs for users. System 200 may choose from the set of components to combine or assemble different components to dynamically generate programs for users. System 200 can also receive the components from experts.

The user interacts with mobile device 224 and views and acts on the goals and nudges. A mobile software application executing on mobile device 224 may include components for personalized coaching 226, social support 228, and gamification 230. Personalized coaching 224 provides coaching advice to the user. Social support 226 facilitates the user's interaction with the other users. The user receives social interaction and social support from team members. Gamification 230 may apply game elements (e.g., point scoring, badges, leaderboards) to the challenges (e.g., programs). A user may interact with gamification elements, such as when the user receives a badge.

System 200 can tailor the delivered content to the ability level of the user, accounting for ability factors such as physical ability and the degree of difficulty, as well as motivational factors such as boredom. The content that mobile device 224 receives from system 200 may include a schedule of activities, including a time for performing activities and descriptions explaining how to perform the activities.

System 200 can tailor intervention communications to the user on a day-to-day basis. For example, if the user finds that walking is too easy, system 200 can immediately assess the situation and intervene by increasing the difficulty of the activity. System 200 may assess the user using questions, sensors, etc. and update user model 210 with information from the user responses and sensor data.

System 200 may use a program provided by a health provider and the radar chart to determine how to advise the user. For example, system 200 may determine that a user is eating appropriately but is not exercising enough. System 200 may then recommend increasing the amount of walking. System 200 can also periodically or randomly checking with the user to determine if the user is following the program and determine if the user's condition has deteriorated or the user has relapsed. System 200 may continuously update the radar chart based on data received about the user, including the user's current condition and progress. System 200 may constantly assesses the user's condition to determine whether to send messages to nudge the user to perform particular actions or activities, or to change his behavior or refrain from performing certain activities.

System 200 can generate the programs (or changes to programs) and coaching recommendations for delivery to mobile device 224 in real-time, or between specific predetermined intervals, or on as needed basis. In some embodiments, system 200 can analyze a program and modify the program to customize a program for the user. System 210 can recommend and adapt a user's next program of activities after the user finishes a program. System 200 may include artificial intelligence techniques to adapt health programs continually for each individual, adjusting to individual progress along different dimensions of their personal health radar. System 200 may also include may include multidimensional recommendation techniques for program activities aimed at maintaining long-term dimensional balance.

In one embodiment, system 200 may interact with the user to determine contextual information and generate content and questions based on the contextual information on a daily basis. For example, the user may report that he performed a 30 minute walk. System 200 may generate questions for the user regarding his 30 minute walk, and follow with the additional questions and/or deliver updated coaching advice immediately. System 200 may provide the user with daily goals and/or activities. For example, system 200 may provide an activity for the user that includes walking 20 minutes or eating three vegetables. System 200 may provide the user with coaching advice, nudges, and reminders while the user is performing activities. For example, system 200 may send a message to a user that his lunch should not exceed a certain number of a certain number of calories and fat or carbohydrates. Since people find it difficult to focus on counting calories and fat, system 200 may send content to the user that focuses on healthy nutritional habits. For example, system 200 may recommend that the user add a vegetable to his lunch, day after day, until the user is eating more vegetables.

As another example, system 200 may send a recommendation to mobile device 224 that the user eats more slowly and mindfully. This affects the calorie intake, but is not directly counting calories. This helps the user to watch his method of eating. System 200 may also recommend that the user not add more salt on top of what he's eating, or suggest that the user read the nutritional label on the food. System 200 thereby incorporates best practices from nutritional experts on better ways to eat.

System 200 can set goals for user and also provide hints and tips to the user to achieve the goals. System 200 may deliver coaching advice to a group of people that are engaged in a team and in the same program together. System 200 may also tailor coaching recommendations to each individual user on the team. System 200 may receive information from multiple team members and perform team-based computations on the server, and system 200 may perform individual-based computations on the server or directly on mobile device 224.

System 200 (e.g., server 119) may deliver messages to a mobile application executing on the user's mobile device. The mobile application receives information from the server and the mobile application displays information to the user, and the user interacts with the mobile application. The user may use mobile device 224 to perform activities with a team of people who are performing the same set of activities. The user may receive personalized coaching from an artificial intelligence agent.

Mobile device 224 (or any other computing device such as web applications running on a personal computer or large displays on home entertainment systems) may display a visual representation of the radar chart. This allows the user to quickly and easily comprehend where the user is with respect to his goals along the five dimensions, and which dimensions the user needs to improve on. System 200 may monitor the user's status with respect to the radar chart along the five dimensions.

Mobile device 224 may include different types of sensors to detect user movement and the surrounding environment. For example, the sensors may include an accelerometer, a gyroscope, a digital compass, a light sensor, a thermometer, a pedometer, a heart rate monitor, a barometer, GPS sensors, and a microphone.

In some embodiments, system 200 include active testing and dynamic programming techniques to learn the expected utilities and costs of different probes, which may vary over time (e.g., individuals may be more receptive while waiting for a train). System 200 may also include machine learning techniques to model and predict human behavior based on active, ongoing assessment of user state and context, including psychosocial variables that may be important to understanding and prediction, and variables that may change dynamically over time (e.g., service satisfaction, mood, receptivity, motivation, attitudes,....)

System 200 can create coaching intervention programs for a particular demographic by combining components, instead of building an application from scratch. The cost of building custom coaching applications for different types of use cases and/or different types of populations can be very high. However, with the component-based approach, system 200 can dynamically combine components to build an application from scratch for a particular demographic. System 200 can adjust the activities of progression and aggression for a particular demographic or community of users.

Note that one can easily build different custom mobile applications based on the platform presented herein. Using the radar chart, the coaching assessment, and the other components as building blocks, one can easily assemble together a mobile application. For example, one can assemble a mobile application for obesity, and another for senior citizens, from the different components. Furthermore, system 200 may also receive data from different data sources, such as from a Fitbit device or a Jawbone device.

In some embodiments, the server can receive configuration information and a request from mobile device 224 and respond by generating customized code and sending code for performing computations to a memory location of mobile device 224 or sending customized code for automatic installation on mobile device 224. This can be code for a custom mobile application. Because the code is located on mobile device 224, mobile device 224 can perform computations even when mobile device 224 is not connected to the Internet.

### Radar Challenge in the NUDG Platform

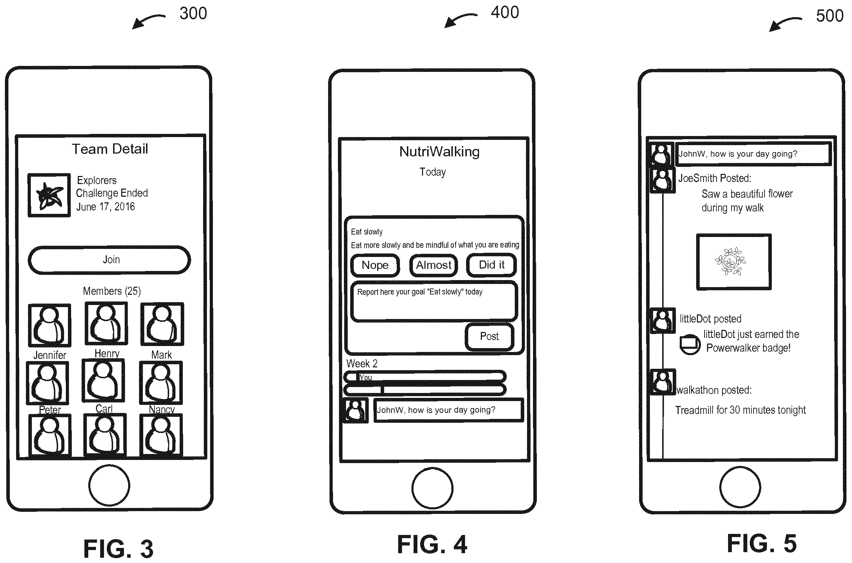

FIG. 3 illustrates an exemplary screenshot 300 of people participating in challenges in small teams. FIG. 4 illustrates an exemplary screenshot 400 of an iPhone application that users may use to report on challenge activities. FIG. 5 illustrates an exemplary screenshot 500 of an iPhone application that users may use to engage with teammates on challenge activities and be social.

NUDG is a software platform that has been developed by PARC that provides challenges (e.g., programs) as defined above. Users may use NUDG through a mobile application installed on their mobile device. When individuals begin using the mobile application, they select a health challenge from an exchange and join a team (e.g., FIG. 3). Each day of the challenge there is a list of activities to do and report on (e.g., FIG. 4). Users also use the mobile application to engage with friends in challenge activities and be social using a team communication stream similar to other social media applications (e.g., FIG. 5). Note that these two figures (e.g., FIG. 4 and FIG. 5) are actually the same view in the mobile application, but one is scrolled down from the other. This is called the Dashboard view of the mobile application. A coaching agent may also use this same team communication stream on the dashboard to provide timely advice, encouragement, and feedback. The system may also deliver messages to teams as iPhone notifications.

### Assumptions

Before designing the specifics of a challenge, challenge designers should determine a goal for the challenge. This should be a concise, coherent, and focused statement about a well-defined set of desired outcomes. For example, one outcome might be to get people to be more physically active so that by the end of 4 weeks they are able to enjoy a 30 minute walk each day, including up and down some small hills or stairs. A related nutrition outcome might to have as a goal that by the end of 8 weeks people know and routinely practice some healthy eating practices, such as eating healthy proteins (e.g., that will aid in the walking goal).

### What is Specified in a Challenge

Subject Matter Experts (SMEs) who know about relevant areas of health and wellness may define the following:

Dimensions: What dimensions of health and wellness are needed for sustained engagement towards intended wellness goals?
- Activities: What activities do people need to do to participate in a challenge?
- Progression/Regression: What sequences of activities are appropriate over time?
- Content/Messaging: What do people need to know to participate in the challenge activities?

### Dimensional Design

This disclosure assumes that the wellness goals have been evaluated and there is evidence that suggests the issue being addressed is indeed a systemic problem, requiring multiple areas of attention to succeed (e.g., as in attaining and keeping a desired level of fitness). Each such area of attention should have a set of activities that promote a beneficial outcome in that area. Note that an activity might likely contribute to more than one area, as yoga can promote mindful meditation that reduces stress as well as develop balance and flexibility. One important point to consider about a dimension is that it is clearly identifiable to the intended audience. It is difficult to ask people to do something they do not care about. If explanations and reasons to do these things are needed, there should be compelling evidence that these activities are a good idea

### (e.g., studies, testimonials, expert reports, etc.)

Selecting the final challenge dimensions is then an iterative process of proposing dimensions and answering basic questions arising from the other attributes of a radar challenge:
- Interchangeable: Can sets of activities by substituted or emphasized or deemphasized within a dimension on a daily or longer-term basis?
- Interleavable: Can sets of activities be substituted or emphasized or deemphasized across dimensions on a daily or longer-term basis?
- Spanning: Is the collection of activities necessary and sufficient to achieve a beneficial outcome?
- Sustainable: Is there sufficient diversity in the activities likely to hold the interest of the intended audiences? Does the intended audience have the ability and resources to engage in a sufficient number of these activities?

### Define Each Activity

A challenge activity is some action that a person needs to do. These activities may happen on a specific day or some number of times per week or as indicated by some other measure (e.g., successful completion of a prerequisite activity). The following information needs to be communicated using one or more of the means described with respect to FIG. 4:
- What is the activity called?
- What detail is needed in explanation for that day for the person to do that activity? A checklist is "who, what, when, where, why, how, how much, how many, how often."
- Give a short summary that gives just enough information to trigger someone to do that activity or prompts them to read more about that activity.

### Arrange Activities in a Schedule

The system may statically schedule activities for specific days and times. Alternatively, the system (or system administrator) may define dynamic progression and regression rules that have the following characteristics:
- Determine an evaluation measure for activity performance, including but not limited to compliance with doing an activity, metric value for activity performance (e.g., metabolic equivalent of task, duration of successfully completed activity in minutes), metric value for activity outcome (e.g., weight lost/gained), subjective value for activity (e.g., self-report for degree of engagement/boredom with an activity).
- Determine partially ordered sequences of activities, including but not limited to assessed degree of difficulty of an activity, assessed degree of interest in an activity (e.g., from crowdsourcing or individual profiles and ratings), amount of resources required for an activity (e.g., need for special
   equipment or training), and equivalence classes for alternative activities that may be substituted one for the other.
- Determine event based rule logic such as in a rule-based system defined by a set of "if-then" statements that uses a set of assertions (e.g., based on evaluation measures above), to which rules on how to act upon those assertions (e.g., progression up or down in the above partial order) are applied.

### Theory-Based Methods for Long-Term Continued Engagement

What might it take to have a person stay engaged with healthy activities in the long run? We are asking this question in the context of a person using some sort of behavior change technology (e.g., a mobile application) to facilitate a healthy lifestyle. Technology acceptance models (TAM) suggest that a computer technology gets used in large part due to its perceived usefulness and its perceived ease of use. These terms are defined as:
- Perceived usefulness (PU): the degree to which a person believes that using a particular system would enhance his or her ... performance.
- Perceived ease-of-use (PEOU): the degree to which a person believes that using a particular system would be free from effort.

Evidence suggests that the perceived usefulness is the stronger effect. Note that TAM and related models were originally defined and studied for work and job performance but have been validated and used in many contexts including health-related processes:
*Perhaps most impressive is that the relationship between PU and intention to use or actual use of health IT is significant in every test, suggesting that to promote use and acceptance, the health IT must be perceived as useful.*

Consider two basic situations for disengagement with a mobile wellness technology:
- a person no longer perceives the application as useful because the need no long exists (e.g., they have reached their goal); or
- a person no longer perceives the application as useful even though the need is still felt but using the application and doing the things it asks is no longer perceived as satisfying activity (e.g., they no longer enjoy the activities or a "plateau" is reached where the body no longer responds to a wellness routine).

Regarding people in the first circumstance, the inventors note from the rebound behavior discussed above that there is likely a misperception about the usefulness of the application that needs addressing. In this case, reconsidering the need is in order. In particular, examining more deeply the rebound behavior one can see that the focus is often seen as getting to a desired level of health and fitness. This process involves participation in a set of unusual circumstances (unusual for the participant).

Trying to hold on to new practices in preference to "normal" life becomes the challenge for the individual. If this is communicated and accepted, then we are in the second disengagement situation - continuing satisfying activities within the mobile application. How might we assess disengagement in this case?

Field trials of prototypes of the mobile application suggests a measure: lack of engagement with healthy activity can be expressed as some degree of "boredom." For example, "I am bored of eating rabbit food," or "I am bored of this exercise routine." In some embodiments, the system can determine the user's degree of engagement with an activity through the user's expressed degree of boredom.

We next address how we define challenges to allow people to deal with boredom sufficiently long enough so that the activities being asked become habitual, or a "new normal"?

### Defining Boredom

Recently, some have proposed a definition of boredom based on a study of the underlying mental processes that occur during an instance of boredom:

### Boredom is the aversive state that occurs when we

• *are not able to successfully engage attention with internal (e.g., thoughts or feelings) or external (e.g., environmental stimuli) information required for participating in satisfying activity,*
• *are focused on the fact that we are not able to engage attention and participate in satisfying activity, and*
• *attribute the cause of our aversive state to the environment.*

For example, consider where someone has become bored with their diet, and states:
*I am bored of eating salads, they are starting to taste like grass. If I have to eat something green one more time I will scream. There is a bacon brie double cheeseburger with cheesy fries on the TV that looks so much better.*

The first statement indicates the non-participation with satisfying action with regards to eating salad, perhaps because of a feeling about an unappetizing taste. The person is not able to attend for some reason to the internal resource (e.g., feeling) of how appetizing a salad could be (e.g., having enjoyed a salad previously). The person is also disengaged from any thoughts about how nutritious a salad actually is and the hunger that it does satisfy. It might also be the case that this person is alone in eating their salad (e.g., the external stimulus of peer participation) and has feelings of being left out (e.g., negative feelings overshadow the positive ones).

The second statement indicates arousal brought on by a perceived monotony and that they have become focused on those negative feelings and not being able to eat what they consider satisfying. The final statement suggests perhaps this aversive state has been triggered or aggravated by actually seeing (e.g., in their local environment) enticing alternatives to what they are currently doing. While it is not likely that instances of boredom would be expressed in such alignment with the definition of boredom, the above demonstrates the different aspects of their aversive state and how it might happen.

### Assessing a Person's Level of Boredom

Based on the above definition, there are several approaches for assessing a person's level of boredom. Consider the following general questions:
- How engaged is attention in the required information?
- What is the level of satisfaction?
- What is the focus of attention?
- Where do we attribute cause of our aversive state?

There are a number of ways of getting answers to these questions:
- The system may ask the user (e.g., self-report, such as question/answer, survey) about where their attention is engaged, level of satisfaction, etc.
- There are also several techniques for inferring engagement. For example, in one implementation of the mobile application there is a history of a person reporting about their activities (e.g., compliance for reporting). Measuring changes in self-efficacy (e.g., belief in one's capacity to execute behaviors necessary to produce specific performance attainments) dynamically may help infer how engaged one's attention is in the required information.

In some implementations, the mobile application may support a conversation stream. Analyzing the contents of this stream may provide estimates of engagement, focus, attribution, or satisfaction (via sentiment analysis) if an individual is questioning, story telling, or complaining about the activities involved.

Studies in workplace boredom suggest a number of reasons for its onset:
- Lack of work or "something to do" (e.g., quantitative underload)
- Dislike of work or task at hand (e.g., qualitative, relevance, lack of meaning)
- Monotony (e.g., repetitiveness or lack of diversity in activity)
- Lack of social engagement (e.g., bored of who you are with)

Understanding causes can lead to specific actionable steps to address emergent issues. In one implementation, interventions in a mobile application (e.g., NUDG mobile application) can be designed to address underload situations, in part by being delivered in a smartphone that is readily available in underload situations.

### Intervening When Boredom is Detected

One may use the above definition for proposing interventions in the mobile application that address instances of boredom. Note that this section primarily discusses "messaging" the user. Messaging means a more general sense of communication and communication design than a single text message communicated to one user. This more general sense of messaging is more fully explored in strategies like communications for behavior change, involving possibly complex interaction and experience design based on understanding of audience and leading to content across different channels of communication. For example, in one implementation (e.g., NUDG mobile application), the following kinds of communication are available for influencing a person:
- Browseable content (e.g., called "cards");
- Text messaging (e.g., daily messaging and coaching tips and instruction delivered by a coaching agent, and question and answer dialogs with the coaching agent);
- Structuring and restructuring of activities a person is offered, where choice of activity (e.g., by the coaching agent) encourages engagement (e.g., to encourage a daily run that a person may have become tired of, assign an activity of taking and posting a "selfie" picture during the run); and
- Indirect messaging in which messaging is used to encourage someone else to message an individual (e.g., getting a peer to encourage a teammate).

Messaging may take many different approaches in encouraging change, including providing information on behavior-health link, providing information about others' approval, providing general encouragement, modeling or demonstrating the behavior, and more. Below are some considerations on the effects that a health and wellness system may have on each attribute of boredom.

### Increase Ability to Engage Attention with Information Required for Participating in Satisfying Activity

One may break this objective down into two issues: engaging attention where needed and encouraging a change in a person's idea of what is "satisfying."
1. Try alternative ways of presenting the information. For example, people remember things better in the context of novelty. One way to do this is tailor the messaging to the audience. Marketing groups go to considerable lengths to train people for this. For example, in the context of a person not being able to attend to the benefits of eating a salad for lunch, one popular technique is to remind them that a salad can involve a number of healthy dressings and additives. Another example is the use of team members sharing posts/media about eating a salad, which could encourage modeling and consequently, adaptation of a healthy behavior.
2. Encourage a change in what "satisfying" means to a person. There are detailed approaches for bringing about changes in attitude (e.g., motivational interviewing). Three themes for messaging for engagement include:
   a. Increase recognition of satisfaction of the activity the user is not participating in. For example, the message "Think of how nice it would be to walk on the beach with your grandchildren when they are old enough" prompts a person to envision a desirable future afforded by the current activity.
   b. Increase recognition of dissatisfaction of not doing the activity the user wants to participate in (e.g., "Eating a heavy meal like garlic fries makes you tired and thirsty afterwards").
   c. Accept or change the perceived unsatisfying nature of the activity in question (e.g., "Walking might be tiring for you now, but it is not causing you harm and this feeling will not last forever").

### Address Focus on Not Being Able to Engage Attention and Participate

If the focus of attention has been placed in an unfruitful direction then the system should change that focus if possible. Distraction can lead to creative states of the brain that foster creativity and problem solving. Studies suggest that loading working memory promotes distraction from negative moods. When the aversive state of boredom is indicated, then possible useful distractions may include:
- Change the focus of attention to something perceived as more pleasant or problem solving (e.g., if the focus is on the greens in the salad, have a person consider other vegetables).
- Encourage talk about the negative feelings (e.g., with teammates in NUDG mobile application) and in particular ways to change the negative feelings (e.g., as in motivational interviewing "change talk").

Such distractors are a tool, but not a guarantee of moving from an aversive state like boredom.

### Address Attribution of Cause of Aversive State to Environment

There are numerous environmental triggers related to unhealthy alternatives such as advertisement by the fast food industry. From the definition of boredom the inventors can identify several basic approaches to breaking out of the effects of environmental influences:
1. Change the perception of the environment. For example, if the trigger of dissatisfaction of having cottage cheese for breakfast again is the leftover pizza still in the refrigerator, then the system might increase the recognition of dissatisfaction that the unhealthy choice brings as discussed above.
2. Change the environment. For example, the system may suggest freezing the leftover pizza as an additional daily activity.
3. Move the attribution to another cause and take steps to address that cause. For example, a conversation involving whether it is the pizza that is proving a distraction or perhaps the lack of support shown by the person who left the pizza there in the first place is proving to be a bigger issue.

### Example Dimensions and Progression/Regression Rules

To illustrate these points, below is listed a set of activities across five dimensions of a radar challenge (e.g., activities relating to aerobic exercise, strength, flexibility, relaxation (and stress reduction), and nutrition). Many other dimensional frameworks are possible and this is one example.

Consider the following list of candidate activities:
- Aerobics
   - Walking (e,g, as described in NutriWalking)
      - Hiking
   - Running (currently undefined)
   - Biking
   - Swimming
   - Snow sports
   - Team sports
   - Aerobic classes (dancing, Zumba etc.)
- Strength
   - Upper Body workouts (e.g., StressBusters and BabySteps)
   - Lower Body workouts (e.g., StressBusters and BabySteps)
   - Core strengthening exercises (e.g., StressBusters and BabySteps)
- Flexibility:
   - Pilates
   - Yoga
   - Tai Chi
   - Dance
   - Stretching (e.g., NutriWalking)
- Relaxation
   - Active Recovery (e.g., StressBusters)
   - Meditation (e.g., BabySteps)
   - Breathing Exercises (e.g., Sleeptastic?)
   - Other exercise (e.g., from other dimensions)
   - Creative activities (e.g., knitting, arts & crafts, music etc.)
- Nutrition
   - Avoid bad/"processed" foods
      - Control sugar
      - Control salt
      - Control fat (e.g., choose healthy fats)
   - Adopt "real" foods
      - Eat a protein (e.g., NutriWalking)
      - Eat a vegetable (e.g., NutriWalking)
      - Portion control
      - Healthy cooking
      - Healthy shopping
      - Hydration
      - Assertiveness (e.g., OptiFast)
      - Dietary supplements (e.g., vitamins etc.)
      - Healthy Eating habits
         - Keeping a food diary (e.g., NutriWalking)
         - Eating slowly (e.g., NutriWalking)

It is also important to coordinate across dimensions to address the following interrelationships as discussed above. The interleavableness of the radar challenge can provide this coordination. Below are examples of inter-dimensional coordination.
- Plan Eating & Exercise
- Strength & Flexibility
- Stress & Eating
- Combined weekly exercise schedule

The next step is to define progression and regression rules for activities within each dimension. The underlying premise on which this definition is based is that within a dimension, progression & regression occur along on one aspect (or axis) first, and then successively or sequentially across the other critical aspects constituting that dimension. Illustrative rules for progression and regression across different aspects within each dimension are described below:
- Aerobics
   - Progression along:
      - MET.mins by varying activity type (Walk → Interval Walk → Walk-Jog/Jog in Interval → Jog)
   - Rules:
      - If success in current level + Rate of Perceived Exertion (RPE) score < 3, then progress to next level on continuum
      - If success in current level + RPE >= 4, then maintain until RPE drops to 3
      - If failure in current level, then drop one level on continuum and assess success & RPE
- Strength
   - Progression along:
      - No. of muscle groups worked out
      - Position in which they are worked out and/or resistance
      - Repetitions & sets
      - Holding duration for isometric exercises
   - Rules:
      - If success in current exercise, then increase reps/sets until fatigue/RPE score < 3
      - After success in increased reps, add muscle groups to exercise (progress to more whole body-type functional movements/exercise). If already wholebody/bodyweight exercise, vary position to increase difficulty or increase resistance
      - If failure in current exercise, unable to complete even 1 rep, then drop to easier to position/reduce muscle groups/reduce resistance
      - If failure in current exercise, able to partially complete required reps, then reduce rep level and maintain until success. Alternatively, reduce resistance but maintain required rep level
- Nutrition
   - Progression along:
      - No. of healthy eating behaviors/activities to be provided in any time window
      - No. of reminders/scaffolding to be provided
      - For applicable behaviors/activities, progress from "easier" to "harder"
   - Rules:
      - Start with one activity at a time, if success in that activity for "x" time, then progress to add another activity simultaneously. Measure and repeat.
      - Measure/observe performance over "y" time on activities/behaviors, remove reminders/scaffolding one at a time and if success, continue to remove reminders every "n" days
      - For activities that can be classified on an easy←→ hard continuum (e.g., eating slowly - start with one meal and progress to all meals; adding one vegetable at a time, one meal at a time); start with easy, if success over "x" time, progress to next level. If failure, then increase reminders for the same level and observe. If success, then gradually reduce reminders as above. If failure even with increased reminders, then drop one level and observe
- Flexibility
   - Progression along:
      - Duration
      - Frequency
      - No. of joints across which muscles are engaged or number of muscle groups engaged or varying position
   - Rules:
      - If success at current duration, increase duration;
      - If success at that, then increase frequency;
      - If success, then include multi-joint activities or increase difficulty by varying position.
      - If failure at current level, then drop one level on multi-joint exercises or positional difficulty, and observe.
      - If failure on that as well, then reduce frequency, and observe
      - If failure on that as well, then reduce duration until success.
- Relaxation
   - Progression along:
      - Duration
      - Frequency
      - Scaffolding/Reminders
      - Type easy←→ hard continuum OR they could all be of equal difficulty
   - Rules:
      - If success on this activity, then progress on duration first, then frequency, then remove reminders/scaffolding gradually
      - If failure on current activity, then reduce duration.
      - If failure after that as well, then reduce frequency.
      - If failure after that as well, add scaffolding/reminders.
      - If failure on a type of activity, then recommend alternative activity based on preference or recency (or lack thereof, i.e., something that was done long ago) → this strategy directly targets/reduces boredom

### Exemplary Process

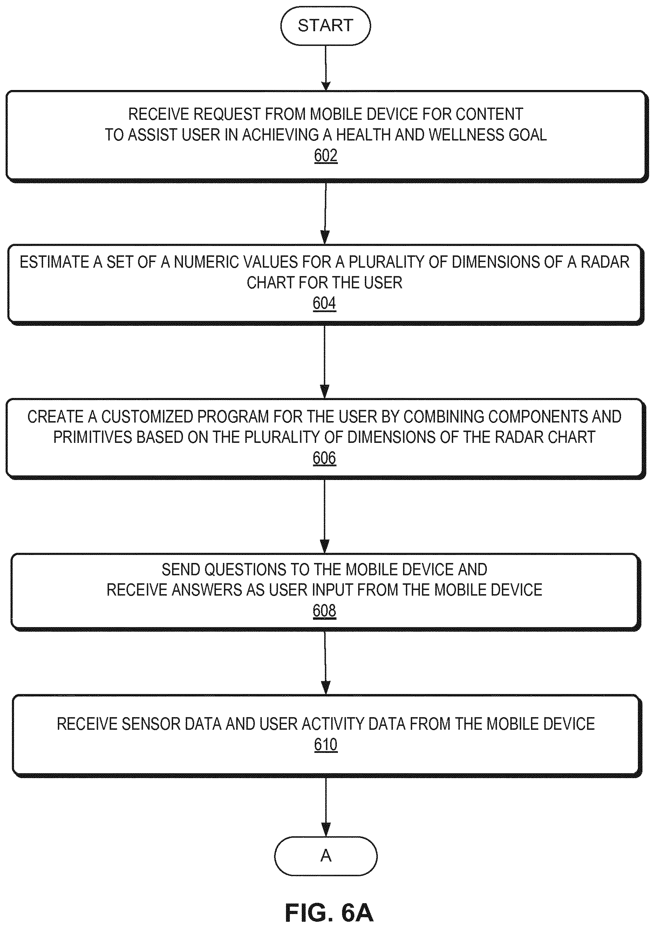

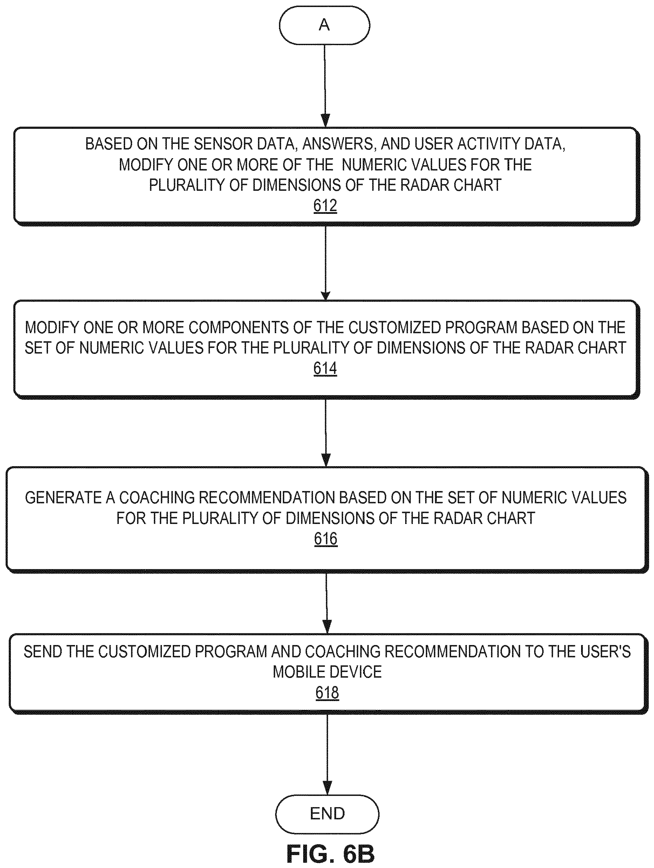

FIG. 6A and 6B presents a flow chart illustrating an exemplary process for delivering a multidimensional wellness coaching program and recommendation to a mobile device (or other computing device) belonging to a user. During operation, the system receives a request from the mobile device for content to assist the user in achieving a health and wellness goal (operation 602). The system then estimates a set of a numeric values for a plurality of dimensions of a radar chart for the user (operation 604). The system may determine the values for the radar chart based on information indicating a current user condition received from the mobile device.

The system may create a customized program for the user by combining components and primitives based on the plurality of dimensions of the radar chart (operation 606). The customized program is created by combining multiple components and/or primitives. The system may deliver the customized program to the mobile device and receive data from the mobile device that the system can use to update the customized program. The system may send questions to the mobile device and receive answers as user input from the mobile device (operation 608). The system may receive sensor data and user activity data from the mobile device (operation 610).

The system may modify one or more of the numeric values for the plurality of dimensions of the radar chart, based on the sensor data, user input, and user activity data (operation 612). The system may modify one or more components of the customized program based on the set of numeric values for the plurality of dimensions of the radar chart (operation 614). The system can continually adapt the customized health coaching program without human authoring or re-authoring effort.

The system may generate a coaching recommendation based on the set of numeric values for the plurality of dimensions of the radar chart (operation 616). The system may then send the customized program and coaching recommendation to the mobile device (operation 618). The user and his teammates may perform the activities that are part of the customized program. The user may report back on completion of activities or tasks.

### Exemplary Apparatus

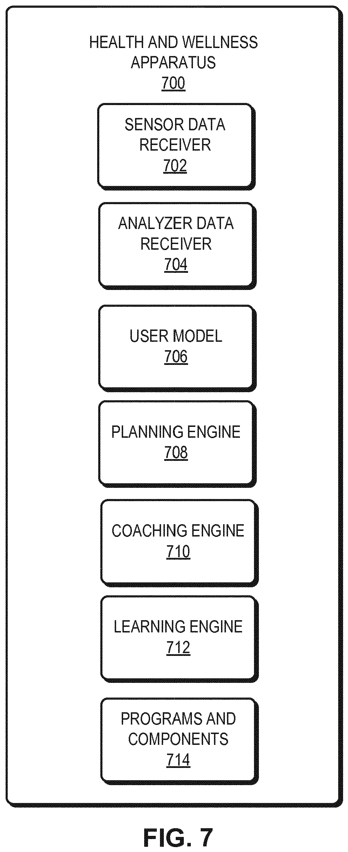

FIG. 7 presents a block diagram illustrating an exemplary health and wellness apparatus 700, in accordance with an embodiment. Apparatus 700 can comprise a plurality of modules which may communicate with one another via a wired or wireless communication channel. Apparatus 700 may be realized using one or more integrated circuits, and may include fewer or more modules than those shown in FIG. 7. Further, apparatus 700 may be integrated in a computer system, or realized as a separate device which is capable of communicating with other computer systems and/or devices.

Specifically, apparatus 700 can comprise a sensor data receiver 702, an analyzer data receiver 704, a user model 706, a planning engine 708, a coaching engine 710, a learning engine 712, and programs and components 714. Note that apparatus 700 may also include additional modules and data not depicted in FIG. 7, and different implementations may arrange functionality according to a different set of modules. Embodiments of the present invention are not limited to any particular arrangement of modules.

Sensor data receiver 702 may receive a continuous stream of sensor data from one or more sensors located on a mobile device. In some embodiments, the system may receive sensor information through a component on the mobile device, such as an activity analyzer. The sensor data indicates motions and actions that the user performs and contextual information for the user, such as the atmospheric pressure.

Analyzer data receiver 704 receives data from the analyzers, including activity, conversation, and interaction data. User model 706 stores information about the user. Planning engine 708 generates goals for delivery to the mobile device based on a health program and information from a user model. Coaching engine 710 generates nudges based on information from the user model. Learning engine 712 learns from the elements received from the analyzers and updates the user model. Programs and components 714 represent the various programs received from providers and constituent components.

### Exemplary Server

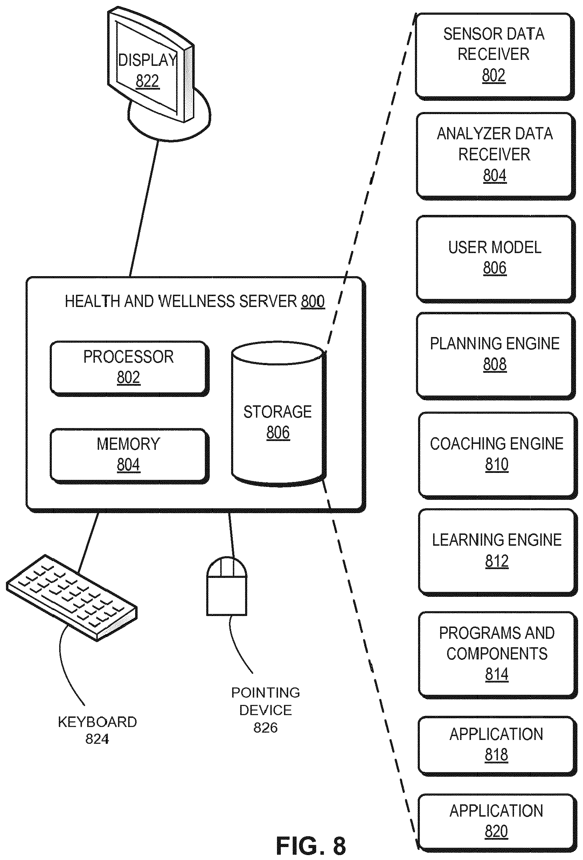

FIG. 8 presents a server 800 in a multidimensional health and wellness system, in accordance with an embodiment of the present invention. In FIG. 8, server 800 includes a processor 802, a memory 804, and a storage device 806. Storage device 806 stores programs to be executed by processor 802 and other data. Specifically, storage device 806 stores a sensor data receiver 802, an analyzer data receiver 804, a user model 806, a planning engine 808, a coaching engine 810, a learning engine 812, and programs and components 814, as well as other applications, such as applications 818 and 820. Health and wellness server 800 may be coupled to an optional display 822, a keyboard 824, and a pointing device 826.

Sensor data receiver 802 may receive a continuous stream of sensor data from one or more sensors located on a mobile device. The sensor data may indicate motions and actions that the user performs and contextual information for the user, such as the atmospheric pressure. Analyzer data receiver 804 receives data from the analyzers, including activity, conversation, and interaction data. User model 806 stores information about the user. Planning engine 808 generates goals for delivery to the mobile device based on a health program and information from a user model. Coaching engine 810 generates nudges based on information from the user model. Learning engine 812 learns from the elements received from the analyzers and updates the user model. Programs and components 814 represent the various programs received from providers and constituent components.

The methods and processes described in the detailed description section can be embodied as code and/or data, which can be stored in a computer-readable storage medium as described above. When a computer system reads and executes the code and/or data stored on the computer-readable storage medium, the computer system performs the methods and processes embodied as data structures and code and stored within the computer-readable storage medium.

Furthermore, the methods and processes described below can be included in hardware modules. For example, the hardware modules can include, but are not limited to, application-specific integrated circuit (ASIC) chips, field-programmable gate arrays (FPGAs), and other programmable-logic devices now known or later developed. When the hardware modules are activated, the hardware modules perform the methods and processes included within the hardware modules.

The foregoing descriptions of embodiments of the present invention have been presented for purposes of illustration and description only. They are not intended to be exhaustive or to limit the present invention to the forms disclosed. Accordingly, many modifications and variations will be apparent to practitioners skilled in the art. Additionally, the above disclosure is not intended to limit the present invention. The scope of the present invention is defined by the appended claims.

## Claims

1. A computer-executable method for delivering a multidimensional wellness coaching program and recommendation to a computing device belonging to a user, comprising:
receiving a request from the computing device for content to assist the user in achieving a health and wellness goal;
estimating a set of a numeric values for a plurality of dimensions of a radar chart for the user;
creating a customized program for the user by combining components and primitives based on the plurality of dimensions of the radar chart, wherein the customized program includes a set of activities for the user to perform;
receiving user input from the computing device;
receiving sensor data and user activity data from the computing device;
based on the sensor data, user input, and user activity data, modifying one or more of the numeric values for the plurality of dimensions of the radar chart;
modifying one or more components of the customized program based on the set of numeric values for the plurality of dimensions of the radar chart;
generating a coaching recommendation based on the set of numeric values for the plurality of dimensions of the radar chart; and
sending the customized program and coaching recommendation to the computing device.

2. The method of claim 1, further comprising generating a suggested goal along a particular dimension for the user using data obtained from a user model and a program received from a provider; and
delivering the suggested goal to the user.

3. The method of claim 1, further comprising displaying a visual representation of the user's health goal at a particular moment in time, including displaying a visual representation of the radar chart that includes five dimensions of strength, nutrition, aerobic, relaxation, and flexibility for the user's current health condition.

4. The method of claim 1, further comprising:
analyzing activities, conversations, and interactions of the user with the teams that the user participates in; and
modifying a user model based on the analysis.

5. A non-transitory computer-readable storage medium storing instructions which when executed by a computer cause the computer to perform a method for delivering a multidimensional wellness coaching program and recommendation to a computing device belonging to a user, the method comprising:
receiving a request from the computing device for content to assist the user in achieving a health and wellness goal;
estimating a set of a numeric values for a plurality of dimensions of a radar chart for the user;
creating a customized program for the user by combining components and primitives based on the plurality of dimensions of the radar chart, wherein the customized program includes a set of activities for the user to perform;
receiving user input from the computing device;
receiving sensor data and user activity data from the computing device;
based on the sensor data, user input, and user activity data, modifying one or more of the numeric values for the plurality of dimensions of the radar chart;
modifying one or more components of the customized program based on the set of numeric values for the plurality of dimensions of the radar chart;
generating a coaching recommendation based on the set of numeric values for the plurality of dimensions of the radar chart; and
sending the customized program and coaching recommendation to the computing device.

6. The storage medium of claim 5, wherein the method further comprises:
generating a suggested goal along a particular dimension for the user using data obtained from a user model and a program received from a provider; and
delivering the suggested goal to the user..

7. The storage medium of claim 5, wherein the method further comprises displaying a visual representation of the user's health goal at a particular moment in time, including displaying a visual representation of the radar chart that includes five dimensions of strength, nutrition, aerobic, relaxation, and flexibility for the user's current health condition.

8. The storage medium of claim 5, wherein the method further comprises:
analyzing activities, conversations, and interactions of the user with the teams that the user participates in; and
modifying a user model based on the analysis.

9. A computing system comprising:
one or more processors;
a memory; and
a computer-readable medium coupled to the one or more processors storing instructions stored that, when executed by the one or more processors, cause the computing system to perform a method for delivering a multidimensional wellness coaching program and recommendation to a computing device belonging to a user, comprising:
receiving a request from the computing device for content to assist the user in achieving a health and wellness goal;
estimating a set of a numeric values for a plurality of dimensions of a radar chart for the user;
creating a customized program for the user by combining components and primitives based on the plurality of dimensions of the radar chart, wherein the customized program includes a set of activities for the user to perform;
receiving user input from the computing device;
receiving sensor data and user activity data from the computing device;
based on the sensor data, user input, and user activity data, modifying one or more of the numeric values for the plurality of dimensions of the radar chart;
modifying one or more components of the customized program based on the set of numeric values for the plurality of dimensions of the radar chart;
generating a coaching recommendation based on the set of numeric values for the plurality of dimensions of the radar chart; and
sending the customized program and coaching recommendation to the computing device.

10. The computing system of claim 9, further comprising
generating a suggested goal along a particular dimension for the user using data obtained from a user model and a program received from a provider; and
delivering the suggested goal to the user.
